# EUROPEAN PATENT SPECIFICATION

(11) **EP 1 925 936 B1**
(45) Date of publication and mention of the grant of the patent: **19.05.2010**
(21) Application number: 06024469.6
(22) Date of filing: 24.11.2006
(51) Int. Cl.: G01N 33/20, G01N 1/12, G01N 25/18

(54) **New thermal analysis device**
Neue Vorrichtung zur thermischen Analyse
Nouveau dispositif d'analyse thermique

(43) Date of publication of application: 28.05.2008
(73) Proprietor: SinterCast AB, 100 55 Stockholm (SE)
(72) Inventor: Popelar, Patrik, 641 53 Katrineholm (SE); Linnarsson, Henrik, 111 52 Stockholm (SE)
(74) Representative: Börlin, Maria

(56) References cited:
- WO-A-99/44022
- WO-A-03/064714
- GB-A- 2 289 758
- US-A1- 2003 043 883
- DATABASE WPI Week 198749 Derwent Publications Ltd., London, GB; AN 1987-347540 XP002432167 & SU 1 308 861 A (CHERP METAL WKS) 7 May 1987 (1987-05-07)

## Description

The present invention provides an improved sampling device for thermal analysis of molten metal, in particular molten cast iron. The invention also provides a kit for such thermal analysis comprising a temperature responsive sensor means and the improved sample device.

### Background of the invention

It is generally accepted in the solidification of metallic alloys that thermal analysis provides an indication of the microstructure with which a given alloy will solidify. This is particularly true of alloys that solidify with two or more distinct phases, such as cast irons, which are comprised of discrete graphite particles in a metallic iron matrix. Depending on the chemical composition and the solidification rate, the morphology of the second phase graphite particles will vary from flake (lamellar) to compacted (vermicular) to nodular (spheroidal). Other intermediate graphite morphologies may also form, and, under certain conditions, the graphite precipitation may be suppressed resulting in the formation of undesirable iron carbides.

By monitoring the latent heat of formation as the graphite particles precipitate and grow, it is possible to deduce the graphite morphology and thus to predict the as-cast microstructure of a given cast iron specimen. Indeed, the time-temperature solidification curves provided by thermal analysis are often referred to as a 'fingerprint' of the cast iron.

In the case of ductile iron, most specifications, particularly for safety-critical components, require that at least 90% of the graphite particles must be present in the form of spheroids (Form VI graphite according to the ISO 945 standard or equivalently, Type I graphite according to the ASTM A-247 standard). In order to achieve the minimum nodularity requirement, most production foundries intentionally overtreat the iron with excess quantities of magnesium (used to modify the shape of the graphite from flake to compacted to spheroidal) and inoculant (used to provide nuclei for the heterogeneous nucleation of the graphite). However, the intentional overtreatment simultaneously creates other potential problems in the production of high quality ductile iron. These include, but are not limited to:
- Incremental magnesium and inoculant additions beyond the minimum requirement unnecessarily increase the production cost. The price of magnesium and inoculant ferroalloys used in the production of ductile iron is typically around EUR 1.50/kg and unnecessary surplus additions may increase the production cost of ductile iron castings by EUR 10 per tonne.
- Increased magnesium and inoculant additions increase the shrinkage tendency of ductile iron and thus require increased feeding to compensate for the shrinkage behaviour. The foundry must select the size of the feeders to compensate for the worst-case condition, ie, when the variation in the foundry process results in the highest magnesium content. At a typical ductile iron sales price EUR 1.50/kg, every 1% improvement in mould yield enabled by reduced feeder size represents a potential savings of EUR 15 per tonne.
- Increased magnesium and inoculant additions reduce the fluidity of the molten iron and increase the potential for mould-filling defects such as misruns and cold-shuts, as well as surface defects related to slag inclusions and dross.
- Increased magnesium and inoculant additions can reduce tool life during subsequent machining operations thus increasing post-processing costs.

In order to improve the production efficiency of ductile iron and specifically, to produce the desired >90% nodularity graphite microstructure with the minimum possible additions of magnesium and inoculant, several researchers have attempted to develop thermal analysis techniques. While the sampling devices advocated by these researchers may provide some information regarding graphite microstructure, the accuracy of these techniques has been hindered by the inherent physical limitations of the sampling device and the sampling technique.

It is evident to the person skilled-in-the-art that the sampling vessel should ideally be neutral and not have any influence on the solidification and thus the development of the graphite microstructure. It is also evident that, because the processing window for the optimal production of ductile iron is so small, the thermal analysis technique must ensure that all variations measured by the thermal analysis are indeed due to differences in the iron, and not due to differences in the sampling technique or sample-to-sample variation.

The thermal analysis sampling devices commonly used in the evaluation of cast iron microstructures are constructed from chemically bonded sand. Some of the obvious shortcomings of these devices that adversely affect the accuracy of microstructure prediction include:
- Sand cups typically have thick sand walls to ensure safe containment of the molten iron. The necessarily thick walls result in a high heat capacity causing the vessel to serve as a heat sink that extracts heat from the iron specimen, thus influencing the solidification behaviour.
- Sand cups, particularly those filled from the open surface of the cup, are liable to variation in the filling technique (oxidation) and sample volume (operator consistency).
- Sand cups typically have open surfaces resulting in large radiation heat losses and thus imbalanced heat losses from the top, sides and bottom of the sample volume.
- Sand cups that rely on coatings to alter the solidification behaviour of the iron (particularly in multiple-cup systems), are affected by the extent of the reaction between the coating and the iron. Different recoveries of the coating into the iron specimen affect the accuracy of the analysis.
- Sand cups that include small amounts of inoculant to alter the solidification behaviour and infer the likely response to inoculant additions during series production are affected by the recovery of the inoculant into the iron sample. The recovery of the inoculant is influenced by a variety of factors including temperature, filling intensity and sample volume that can each affect the accuracy of the analysis.
- The thermocouples found in sand cups are rigidly mounted and consumed with each analysis. Therefore, variations in thermocouples directly influence the accuracy of the thermal analysis.

One example of a sampling device for thermal analysis is described in GB2289758. The device is made of refractory material and comprises two sampling cavities having thick walls. In each cavity is a thermocouple arranged. In use, melt is first poured into the first cavity and then flows over a partition wall to the second cavity when the first cavity is full.

A further example of a sampling device for thermal analysis is shown in WO9944022. The device comprises a mould consisting of refractory material. A cylindrical duct is arranged in the top of the mould for receiving molten metal. The duct connects to a spherical cavity which is connected to a cylindrical cavity. The cavities have thick walls. A thermocouple is arranged in the spherical cavity and one thermocouple is arranged in the transition zone between the spherical cavity and the cylindrical part.

Accordingly, there is a need for improved thermal analysis sampling devices

### Summary of the invention

The invention provides an improved sampling device for thermal analysis of molten metal, and in particular ductile cast iron. The sampling device is intended to be filled with liquid metal to be analysed, and accordingly, it is a container having an upper side and a lower side. The sampling device has a common filling inlet on the upper side. Furthermore, the device comprises at least two cavities. Each of these cavities has a protective tube adapted for enclosing a temperature responsive sensor member. Moreover, the common filling inlet is branched into at least two filling channels ending in said cavities.

Any type of temperature responsive sensor member that is suitable for measuring temperatures in molten cast iron could be used in connection with the present invention. An example of such members is a thermocouple.

According to the present invention, the cavities may have different sizes. It is especially preferred that the volume of the largest cavity is at least twice as large as the volume of the smallest cavity. Furthermore, it is preferred that the cavities are at least partially spherical.

It is preferred that there is a minimum of thermal connection between the cavities. One way of obtaining such minimal thermal connection is to locate the branching point of filling inlet above the cavities. Furthermore, it is preferred to equip each cavity with an overflow outlet on top of the cavity thereby preventing a surplus of molten metal to remain in the filling channels and the filling inlet.

The sampling device is manufactured of a material chosen from the group of steel and a moulded fibrous refractory cloth material.

Finally, the invention provides a kit of parts intended for thermal analysis of solidifying metal, said kit comprising:
a) a temperature responsive sensor means; and
b) a sampling device as outlined above.

### Detailed description of the invention

The present invention has been specifically developed to overcome the inherent physical limitations of sand cups and to provide a stable platform for the thermal analysis of ductile iron. The features of the novel sampling device are described in relation to the enclosed figures, in which
Fig. 1 discloses a side view of a sampling device according to the present invention;
Fig. 2 discloses a view from above of the sampling device in Fig. 1; and
Fig. 3 discloses a side view of the sampling device in Fig. 1, rotated 90 °.

It is well known that the graphite microstructure in ductile iron is influenced by the solidification rate, with higher solidification rates resulting in the formation of more, smaller, and generally better formed nodules. The sampling device proposed in the current invention therefore principally consists of two discrete spheroidal chambers to exploit the cooling rate effect. The two chambers - of which the volume of the larger chamber is approximately four times greater than that of the smaller chamber - provide two different, but consistent and controlled, solidification conditions. In this way, the two different conditions provide different thermal analysis fingerprints that can be compared and contrasted to resolve the features of the graphite microstructure. In comparison to the use of coatings or inoculant additions to impose different solidification conditions, the volume of the two spheroidal sampling chambers can be steadfastly relied upon to always yield consistent sampling conditions and is not prone to the consistency of recovery.

The present invention also incorporates a series of other novel features that ensure consistent sampling conditions. These features are summarised as follows:
1. The use of fully enclosed spheroidal sample chambers prevents radiation heat loss from an otherwise open metal surface and ensures equal heat loss in all directions, thus providing the simplest geometry for uniform and consistent solidification.
2. The thin walls of the device have low thermal mass and are constructed of a material with low heat capacity to ensure a rapid establishment of thermal equilibrium between the sample mass and the sampling vessel. This provides high thermal reproducibility and minimises the influence of the sampling vessel on the development of the solidification.
3. The sampling device is free-standing to ensure uniform heat losses in all directions and to minimise conductive heat losses
4. The overflow outlet allows the iron in the filling channels to drain once the spheroidal chambers have become filled. This ensures consistent fill-volumes and prevents any thermal connection between the two chambers. The overflow outlet also ensures that the feeding channels do not remain filled to provide a thermal bridge between the two sample chambers.
5. The use of a common filling point ensures constant sample-to-sample volume. The common filling also allows both samples to be obtained with a single filling action thus providing operational convenience.
6. The sampling device utilises reusable thermocouples that are located within protective tubes. These thermocouples are extracted after each analysis. The hot junctions of the thermocouples are strategically located in the thermal centres of the spheroidal chambers. The use of reusable thermocouples improves the sample-to-sample consistency relative to conventional thermal analysis devices that rely upon single-use consumable thermocouples.

The construction of the described sampling device can be achieved in a variety of ways. In one embodiment, the vessel can be constructed from a moulded fibrous refractory cloth material that has been impregnated by any one of a number of hardening or binder agents known in the foundry industry. In another embodiment, the device can be constructed from two embossed steel sheets that are welded or crimped together. Both embodiments can provide high dimensional and thermal reproducibility combined with ease of manufacture and low production cost. One added advantage of the steel embodiment is that the finished samples can be directly recycled within the foundry by remelting in the standard foundry charge mix.

It is possible to alter the thermal conditions within the spheroidal chambers by decoupling the thermal communication between the sampling device and its local environment. This can practically be achieved by cladding or blanketing the sampling device with materials of differing insulation efficiency, by surrounding the sampling device in an enclosure, or by any other mechanical solution to establish a Dewar-type insulation. Such actions may be beneficial, for example, to adapt the sampling vessel conditions to emulate the production of large ductile iron castings with slow solidification rates.

The present invention provides consistent sampling conditions to enable an accurate thermal analysis using interpretation methods known per se to determine the graphite microstructure. Suitable such methods are disclosed in WO 99/25888, WO 00/37698 and WO 00/37699. This ability enables the foundry to reliably achieve the minimum 90% nodularity requirement for ductile iron components while using minimum amounts of magnesium and inoculant. Ultimately, the subject of the present invention enables improved control of the ductile iron production process and provides improved process efficiency and cost effectiveness.

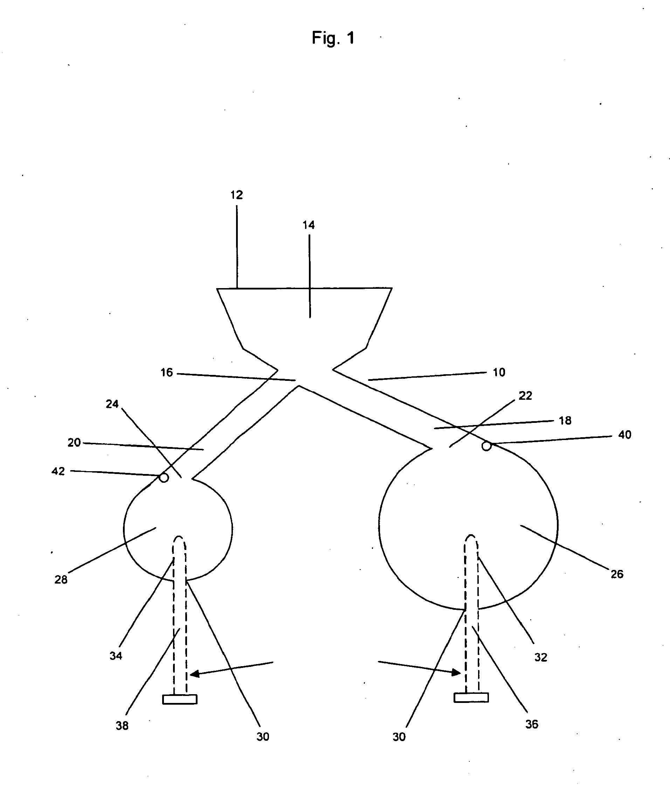

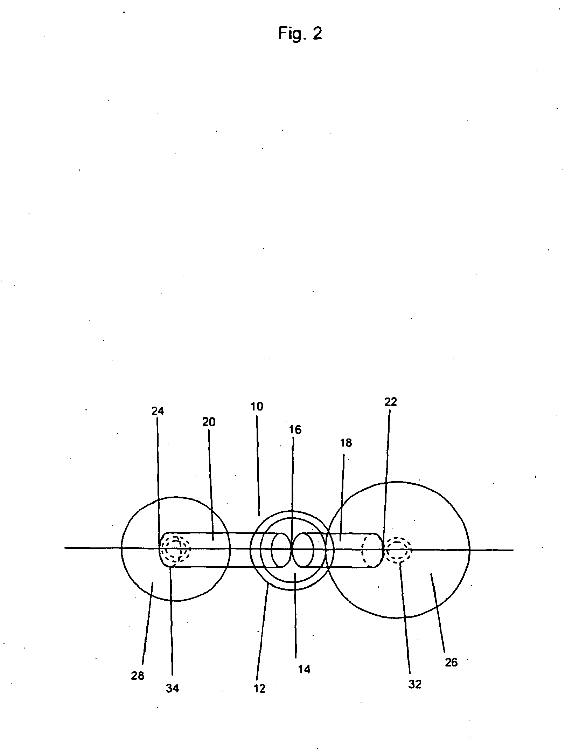

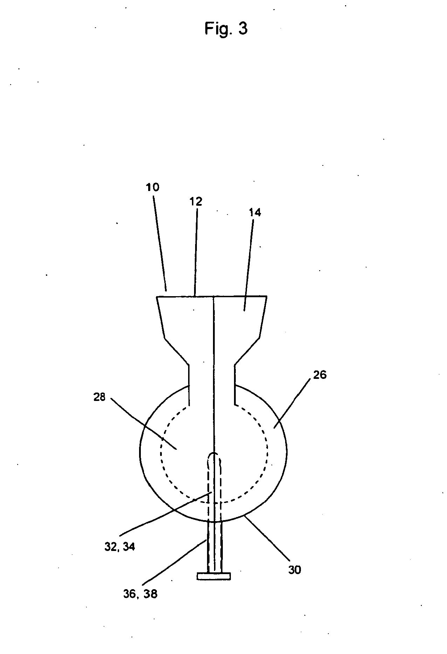

Referring to figures 1, 2, and 3, the sampling device 10 has an upper side 12 and a lower side 30. There is a common filling inlet 14 on the upper side 12 of the sample device 10. There are two cavities, a larger cavity 26 and a smaller cavity 28. Each cavity 26, 28 has a protective tube 32, 34 adapted for enclosing a temperature responsive sensor member 36, 38. The common filling inlet 14 is branched at a branching point 16 into two filling channels 18, 20, ending at ending points 22, 24 on the upper side of the cavities 26, 28. The branching point 16 is located above cavities 26, 28. Finally, there are overflow outlets 40, 42 close to the ending points 22, 24 of the filling channels 18, 20 in order to prevent molten metal from remaining in the common filling inlet 14 and the filling channels 18, 20 when the cavities 26, 28 have been filled with molten metal.

## Claims

1. A sampling device (10) for thermal analysis of molten metal, in particular molten cast iron, said sampling device (10) being intended to be filled with liquid metal to be analysed, said sampling device (10) being a container having an upper side (12) and a lower side (30), said container comprising one common filling inlet (14) on the upper side of said container and at least two cavities (26, 28), each cavity (26, 28) having a protective tube (32, 34) adapted for enclosing a temperature responsive sensor member (36, 38), **characterised in that** said common filling inlet (14) is branched into at least two filling channels (18, 20) ending in said cavities (26, 28), wherein the sampling device (10) is manufactured of a material chosen from the group of steel and a moulded fibrous refractory cloth material.

2. A sampling device according to claim 1, **characterised in that** said cavities have different sizes.

3. A sampling device according to claim 2, **characterised in that** the volume of the largest cavity (26) is at least twice as large as the volume of the smallest cavity (28).

4. A sampling device according to anyone of claims 1 - 3, **characterised in that** the cavities (26, 28) are at least partially spherical.

5. A sampling device according to anyone of claims 1 - 4, **characterised in that** there is a minimum of thermal connection between the cavities (26, 28).

6. A sampling device according claim 5, **characterised in that** the branching point (16) of the filling inlet (14) is located above the cavities (26, 28).

7. A sampling device according to anyone of claims 1 - 6, **characterised in that** each cavity (26, 28) is equipped with an overflow outlet (40, 42).

8. A kit of parts intended for thermal analysis of solidifying metal, said kit comprising:
a) a temperature responsive sensor means; and
b) a sampling device according to anyone of claims 1 - 7.

## Patentansprüche

1. Probennahmevorrichtung (10) zur thermischen Analyse von geschmolzenem Material, insbesondere von geschmolzenem Gusseisen,
wobei die Probennahmevorrichtung (10) dazu gedacht ist, um mit zu analysierendem flüssigem Metall gefüllt zu werden,
wobei die Probennahmevorrichtung (10) ein Behälter ist, der eine Oberseite (12) und eine Unterseite (30) aufweist,
wobei der Behälter einen gemeinsamen Fülleinlass (14) auf der Oberseite des Behälters und zumindest zwei Aushöhlungen (26, 28) aufweist,
wobei jede Aushöhlung (26, 28) eine geschützte Röhre (32, 34) aufweist, die zum Umschließen eines auf Temperatur ansprechenden Sensorelementes (36, 38) ausgebildet ist,
**dadurch gekennzeichnet, dass**
der gemeinsame Fülleinlass (14) in zumindest zwei Füllkanäle (18, 20) geteilt ist, die in den Aushöhlungen (26, 28) enden,
wobei die Probennahmevorrichtung (10) aus einem aus der Gruppe aus Stahl und geformtem, faserförmigem, hitzebeständigem Gewebematerial ausgewählten Material hergestellt ist.

2. Probennahmevorrichtung nach Anspruch 1, **dadurch gekennzeichnet, dass** die Aushöhlungen unterschiedliche Größen aufweisen.

3. Probennahmevorrichtung nach Anspruch 2, **dadurch gekennzeichnet, dass** das Volumen der größten Aushöhlung (26) zumindest zweimal so groß ist wie das Volumen der kleinsten Aushöhlung (28).

4. Probennahmevorrichtung nach einem der Ansprüche 1 bis 3, **dadurch gekennzeichnet, dass** die Aushöhlungen (26, 28) zumindest teilweise kugelförmig sind.

5. Probennahmevorrichtung nach einem der Ansprüche 1 bis 4, **dadurch gekennzeichnet, dass** eine minimale thermische Verbindung zwischen den Aushöhlungen (26, 28) vorliegt.

6. Probennahmevorrichtung nach Anspruch 5, **dadurch gekennzeichnet, dass** der Verzweigungspunkt (16) des Fülleinlasses (14) über den Aushöhlungen (26, 28) angeordnet ist.

7. Probennahmevorrichtung nach einem der Ansprüche 1 bis 6, **dadurch gekennzeichnet, dass** jede Aushöhlung (26, 28) mit einer Überlauföffnung (40, 42) versehen ist.

8. Teilesatz, der zur thermischen Analyse von erstarrendem Material gedacht ist, wobei der Satz umfasst:
a) eine auf Temperatur ansprechende Sensoreinrichtung und
b) eine Probennahmevorrichtung nach einem der Ansprüche 1 bis 7.

## Revendications

1. Dispositif de prélèvement d'échantillons (10) destiné à une analyse thermique d'un métal fondu, en particulier de la fonte fondue, ledit dispositif de prélèvement d'échantillons (10) étant prévu pour être rempli de métal liquide à analyser, ledit dispositif de prélèvement d'échantillons (10) étant un contenant qui présente un côté supérieur (12) et un côté inférieur (30), ledit contenant comprenant une entrée de remplissage commune (14) située sur le côté supérieur dudit contenant et au moins deux cavités (26, 28), chaque cavité (26, 28) présentant un tube de protection (32, 34) adapté pour enfermer un élément de capteur sensible à la température (36, 38), **caractérisé en ce que** ladite entrée de remplissage commune (14) se ramifie en au moins deux canaux de remplissage (18, 20) qui aboutissent dans lesdites cavités (26, 28), dans lequel le dispositif de prélèvement d'échantillons (10) est réalisé dans un matériau sélectionné dans le groupe constitué par un acier et un matériau de tissu réfractaire fibreux moulé.

2. Dispositif de prélèvement d'échantillons selon la revendication 1, **caractérisé en ce que** lesdites cavités présentent des tailles différentes.

3. Dispositif de prélèvement d'échantillons selon la revendication 2, **caractérisé en ce que** le volume de la plus grande cavité (26) est au moins deux fois plus grand que le volume de la plus petite cavité (28).

4. Dispositif de prélèvement d'échantillons selon l'une quelconque des revendications 1 à 3, **caractérisé en ce que** les cavités (26, 28) sont au moins en partie sphériques.

5. Dispositif de prélèvement d'échantillons selon l'une quelconque des revendications 1 à 4, **caractérisé en ce qu'**il y a un minimum de connexion thermique entre les cavités (26, 28).

6. Dispositif de prélèvement d'échantillons selon la revendication 5, **caractérisé en ce que** le point de ramification (16) de l'entrée de remplissage (14) se situe au-dessus des cavités (26, 28).

7. Dispositif de prélèvement d'échantillons selon l'une quelconque des revendications 1 à 6, **caractérisé en ce que** chaque cavité (26, 28) est dotée d'une sortie de trop-plein (40, 42).

8. Ensemble d'éléments prévu pour une analyse thermique d'un métal en cours de solidification, ledit ensemble comprenant :
a) des moyens de capteur sensibles à la température ; et
b) un dispositif de prélèvement d'échantillons selon l'une quelconque des revendications 1 à 7.
